# EUROPÄISCHE PATENTSCHRIFT

(11) **EP 0 405 000 B1**
(45) Veröffentlichungstag und Bekanntmachung des Hinweises auf die Patenterteilung: **09.11.1994**
(21) Anmeldenummer: 89111994.3
(22) Anmeldetag: 30.06.1989
(51) Int. Cl.: A61B 6/04

(54) **Patientenlagerungstisch mit einer mit einem Ausschnitt versehenen Lagerungsplatte**
Patient-supporting table having a supporting plate provided with a recess
Table de support pour patients à plateau de support muni d'une découpure

(43) Veröffentlichungstag der Anmeldung: 02.01.1991
(73) Patentinhaber: SIEMENS AKTIENGESELLSCHAFT, 80333 München (DE)
(72) Erfinder: Schäfer, Willi, Dipl.Ing.(FH), D-8520 Erlangen (DE)

(56) Entgegenhaltungen:
- EP-A- 0 294 679
- DE-A- 3 222 332
- FR-A- 1 457 013
- FR-A- 2 013 178
- State of the Art Extracorporal Shock Wave Lithotripsy, L.B. Kandel, L.H. Harrison, D.L. McCullough, Futura Publishing Company, Inc., Mount Kisko, New York, 1987, Seiten 84 und 85

## Beschreibung

Die Erfindung betrifft einen Patientenlagerungstisch mit einer Lagerungsplatte, welche einen Ausschnitt aufweist, durch den die Körperoberfläche eines auf der Lagerungsplatte liegenden Patienten für medizinische Zwecke zugänglich ist, wobei der Ausschnitt als Ausnehmung in einer der Begrenzungskanten der Lagerungsplatte ausgeführt ist.

Solche Patientenlagerungstische werden für die unterschiedlichsten Anwendungen benötigt, bei denen es erforderlich ist, daß die Körperoberfläche eines Patienten durch den Ausschnitt zugänglich ist, sei es um einen Eingriff vornehmen zu können oder um ein medizinisches Gerät, z.B. einen Lithotripter, an die Körperoberfläche des Patienten applizieren zu können.

Ein Patientenlagerungstisch mit einem Ausschnitt ist in dem Prospekt der Fa. Siemens, "LITHOSTAR-Universeller urologischer Arbeitsplatz für Therapie und Diagnostik", Druckzeichen: A91001-M1027-G490-01 PA 4864, sowie in der EP-A-0 294 679, beschrieben. Bei dem bekannten Patientenlagerungstisch ist ein unterhalb der Lagerungsplatte angeordnetes Stoßwellenrohr vorgesehen, das zur Zertrümmerung von im Körper des Patienten befindlichen Konkrementen, z.B. Nierensteinen, fokussierte Stoßwellen abgibt. Zur Behandlung wird das Stoßwellenrohr durch den als rechteckige Öffnung ausgeführten Ausschnitt der Lagerungsplatte mit der Körperoberfläche eines auf der Lagerungsplatte liegenden Patienten in Eingriff gebracht und derart akustisch an die Körperoberfläche des Patienten angekoppelt, daß sich das zu zertrümmernde Konkrement im Brennpunkt der Stoßwellen befindet. Unter der Einwirkung einer Serie von mittels des Stoßwellenrohres erzeugter fokussierter Stoßwellen zerfällt das Konkrement in Bruchstücke, die auf natürlichem Wege abgehen können. Infolge der beschriebenen Ausbildung des Ausschnittes ist nur ein geringer Teil der sich im Bereich des Ausschnittes befindlichen Körperoberfläche des Patienten zugänglich. Da der Patient im Interesse einer stabilen Positionierung relativ zu dem Fokus der Stoßwellen entweder nur in Bauch- oder Rückenlage gelagert werden kann, kann die Einstrahlung von Stoßwellen nur frontal oder dorsal erfolgen.

Hier könnte durch eine Ausbildung des Patientenlagerungstisches gemäß der FR-A-2 013 178 Abhilfe geschafft werden. Im Falle dieses Patientenlagerungstisches ist die Lagerungsplatte in zwei getrennte Abschnitte unterteilt, die demnach einen sich über die gesamte Breite der Lagerungsplatte erstreckenden Spalt begrenzen. Die beiden Abschnitte der Lagerungsplatte sind durch ein Gestänge miteinander verbunden.

Abhilfe bietet auch der in "State of the Art Extracorporeal Shock Wave Lithotripsy", 1987, Seiten 84 und 85, L.B. Kandel, L.H. Harrison, D.L. McCullough; "Alternative Lithotripters", beschriebene Patientenlagerungstisch. Bei diesem ist der Ausschnitt als Ausnehmung in einer der Begrenzungskanten der Lagerungsplatte ausgeführt. Infolge dieser Ausbildung des Ausschnittes ist die mit der Ausnehmung versehene Begrenzungskante der Lagerungsplatte unterbrochen, so daß sich eine deutlich verbesserte Zugänglichkeit zur Körperoberfläche des Patienten ergibt. Die Gestalt der Ausnehmung kann den jeweiligen Bedürfnissen entsprechend ausgeführt werden. Im einfachsten Fall kann eine rechteckförmige Ausnehmung vorgesehen sein. Die Erstreckung der Ausnehmung in Richtung der mit der Ausnehmung versehenen Begrenzungskante der Lagerungsplatte ist ebenso wie die Erstreckung der Ausnehmung quer zu der genannten Begrenzungskante so zu wählen, daß eine sichere Lagerung des Patienten und eine ausreichende mechanische Stabilität der Lagerungsplatte gewährleistet sind. Insbesondere müssen die Abmessungen der Ausnehmung derart gewählt sein, daß die Gefahr eines Durchrutschens des Patienten durch die Ausnehmung nicht besteht. Es ist von Vorteil, die Ausnehmung an einer solchen Stelle der Begrenzungskante der Lagerungsplatte vorzusehen, daß der Körper des Patienten bei ordnungsgemäßer Lagerung im Bereich der Begrenzungskanten der Ausnehmung durch die Lagerungsplatte ausreichend unterstützt ist.

Der Erfindung liegt die Aufgabe zugrunde, einen Patientenlagerungstisch der eingangs genannten Art so auszubilden, daß durch den Ausschnitt ein möglichst großer Teil der sich im Bereich des Ausschnittes befindlichen Körperoberfläche des Patienten für medizinische Zwecke zugänglich ist und insbesondere ein medizinisches Gerät durch den Ausschnitt an die Körperoberfläche des Patienten applizierbar ist und die verbesserte Zugänglichkeit für beide Seiten des Patienten gegeben ist.

Nach der Erfindung wird diese Aufgabe dadurch gelöst, daß der Patientenlagerungstisch ein Basisteil aufweist, an dem die Lagerungsplatte anbringbar ist, und daß die Lagerungsplatte zweiteilig ausgeführt ist, wobei ein Plattenabschnitt die Ausnehmung aufweist und mit dem anderen Plattenabschnitt wahlweise derart verbindbar ist, daß sich die Ausnehmung auf der einen oder der anderen Seite des Patientenlagerungstisches befindet. Zur Änderung der Position der Ausnehmung muß also lediglich der mit der Ausnehmung versehene Plattenabschnitt gewendet werden, was leicht zu bewerkstelligen ist. Dabei ist es von Vorteil, wenn gemäß einer Variante der Erfindung die Lagerungsplatte bzw. der die Ausnehmung aufweisende Plattenabschnitt zu einer quer zu der mit der Ausnehmung versehenen Begrenzungskante durch die Ausnehmung verlaufenden Symmetrieachse spiegelsymmetrisch ausgebildet ist, da dann die Lage der Ausnehmung in Richtung der die Ausnehmung aufweisende Begrenzungskante für beide Positionen die gleiche ist.

Eine bevorzugte Ausführungsform der Erfindung sieht vor, daß eine der Längskanten der Lagerungsplatte mit der Ausnehmung versehen ist. In diesem Falle ist die Erstreckung der Ausnehmung quer zu der mit der Ausnehmung versehenen Längskante der Lagerungsplatte praktisch nur durch die im Bereich der Ausnehmung aus Gründen der mechanischen Stabilität der Lagerungsplatte erforderliche Restbreite der Lagerungsplatte begrenzt. Außerdem ist eine gute laterale Zugänglichkeit zu dem Körper des Patienten gewährleistet, ohne daß dieser die instabile Seitenlage einnehmen muß.

Eine Ausführung der Erfindung sieht vor, daß der Patientenlagerungstisch ein freies, d.h. nicht unterstütztes, Ende aufweist und mit seinem anderen Ende an einem Tragteil angebracht ist. Der Patientenlagerungstisch ist also freitragend ausgebildet, so daß der Patientenlagerungstisch selbst bzw. ein auf diesem liegender Patient gut zugänglich ist. Im Falle dieser Ausführung wird der Patientenlagerungstisch vorzugsweise mit seinem Basisteil an dem Tragteil, z.B. einem Sockel oder einem Stativ, befestigt.

Im Interesse einer erhöhten mechanischen Stabilität des Patientenlagerungstisches sieht eine Variante der Erfindung vor, daß ein Tragholm zur Unterstützung der Lagerungsplatte vorgesehen ist, der sich wenigstens im Bereich der Ausnehmung entlang der der Ausnehmung gegenüberliegenden Begrenzungskante der Lagerungsplatte erstreckt. Für den Fall eines freitragenden Patientenlagerungstisches ist gemäß einer Ausführungsform der Erfindung vorgesehen, daß der Tragholm ein freies Ende aufweist, an dem ein sich quer zur Längsachse des Tragholmes erstreckender Tragarm anbringbar ist, der die Lagerungsplatte im Bereich des freien Endes des Patientenlagerungstisches unterstützt.

Um auch Lagerungsplatten, deren Ausnehmung sich auf der einen oder der anderen Seite des Patientenlagerungstisches befinden kann, in der beschriebenen Weise unterstützen zu können, ist gemäß einer Ausführungsform der Erfindung vorgesehen, daß zwei Tragholme vorgesehen sind, die in Abhängigkeit von der Position der Ausnehmung auf der einen oder der anderen Seite des Patientenlagerungstisches wahlweise ausziehbar sind. Der Tragarm ist dann vorzugsweise derart ausgebildet, daß er wahlweise an dem einen oder dem anderen Tragholm angebracht werden kann.

Gemäß einer Variante der Erfindung ist vorgesehen, daß die Tragholme in dem Basisteil ausziehbar aufgenommen sind.

Ein Ausführungsbeispiel der Erfindung wird nachfolgend anhand der beigefügten Zeichnungen erläutert. Es zeigen:
- Fig. 1: eine perspektivische Ansicht eines nicht die Erfindung verkörpernden Patientenlagerungstisches,
- Fig. 2: einen Querschnitt durch den Patientenlagerungstisch entsprechend der Linie II-II in Fig. 1,
- Fig. 3: einen Längsschnitt durch den Patientenlagerungstisch entsprechend der Linie III-III in Fig. 1,
- Fig. 4: eine perspektivische Darstellung der Einzelheit A gemäß Fig. 1 bei abgenommener Lagerungsplatte,
- Fig. 5: eine perspektivische Ansicht des Patientenlagerungstisches gemäß Fig. 1 bei gewendeter Lagerungsplatte,
- Fig. 6: eine perspektivische Ansicht eines erfindungsgemäßen Patientenlagerungstisches, und
- Fig. 7: eine perspektivische Ansicht der Einzelheit B gemäß Fig. 6 in Form einer Explosionsdarstellung.

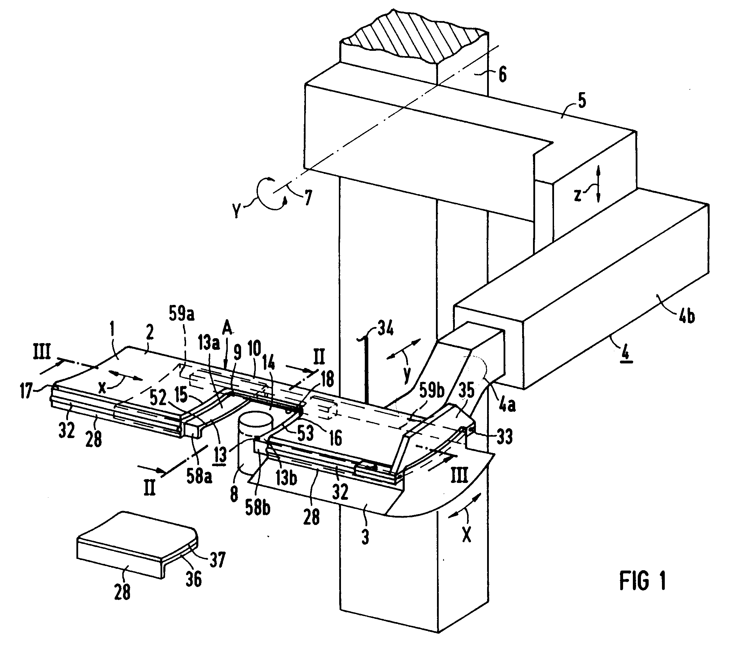

Die Fig. 1 bis 5 zeigen einen nicht gemäß der Erfindung ausgebildeten Patientenlagerungstisch, der Bestandteil eines Arbeitsplatzes zur Behandlung eines Patienten mit fokussierten Stoßwellen, beispielsweise zur nicht-invasiven Zertrümmerung von im Körper des Patienten befindlichen Konkrementen wie Nieren- oder Gallenblasensteinen, ist. Die fokussierten Stoßwellen werden mittels einer Stoßwellenquelle, wie sie beispielsweise in der DE-OS 33 28 051 beschrieben ist, erzeugt und in den Körper eines zu behandelnden Patienten eingeleitet, indem die Stoßwellenquelle mit ihrem durch einen flexiblen Balg gebildeten Applikationsende mit der Körperoberfläche des Patienten derart in Eingriff gebracht wird, daß eine akustische Koppelung vorliegt. Dabei werden der Patient und die Stoßwellenquelle durch entsprechendes Verstellen des Patientenlagerungstisches und der Stoßwellenquelle relativ zueinander derart positioniert, daß sich das zu zertrümmernde Konkrement im Fokus der Stoßwellen befindet. Unter der Wirkung einer Vielzahl von Stoßwellen zerfällt das Konkrement in feine Bruchstücke, die auf natürlichem Wege vom Patienten ausgeschieden werden können.

Der Patientenlagerungstisch besitzt eine Lagerungsplatte 1 mit einer Auflagefläche 2 für den Patienten. Außerdem ist ein Basisteil 3 vorgesehen, das die Lagerungsplatte 1 trägt, wobei die Lagerungsplatte 1 von dem Basisteil 3 getrennt werden kann.

Der Patientenlagerungstisch ist mit seinem Basisteil 3 derart mittels geeigneter, nicht dargestellter Gelenkmittel an einem Tragteil 4 angebracht, daß er um eine parallel zu seiner Längsachse verlaufende Achse schwenkbar ist, so wie dies durch den gekrümmten Doppelpfeil X angedeutet ist. Das Tragteil 4 weist zwei teleskopartig ineinander gefügte Armteile 4a, 4b auf, so daß der Patientenlagerungstisch quer zur Richtung seiner Längsachse in einer parallel zur Auflagefläche 2 verlaufenden Ebene in Richtung des Doppelpfeiles y verstellbar ist. Das Tragteil 4 ist mit seinem Armteil 4b an einem armförmigen Träger 5 mittels geeigneter, nicht dargestellter Führungsmittel derart befestigt, daß der Patientenlagerungstisch rechtwinklig zur Ebene seiner Lagerungsplatte 1 verstellbar ist, so wie dies durch den Doppelpfeil z angedeutet ist. Der Träger 5 ist an einem Ständer 6 um eine quer zur Längsachse des Patientenlagerungstisches und parallel zur Ebene der Lagerungsplatte 1 verlaufende Achse 7 mittels eines geeigneten, nicht dargestellten Gelenkes in Richtung des Doppelpfeiles Y schwenkbar. Außerdem ist die Lagerungsplatte 1 relativ zu dem Basisteil 3 in Richtung der Längsachse des Patientenlagerungstisches geradlinig verstellbar, so wie dies durch den Doppelpfeil x angedeutet ist.

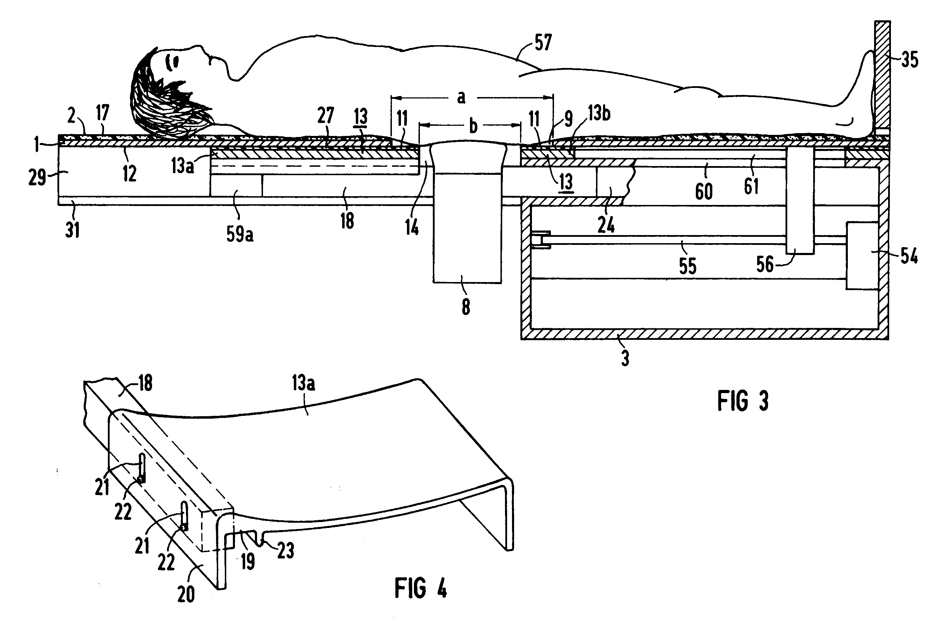

Um die beschriebenen Verstellbewegungen ausführen zu können, sind nicht dargestellte Elektromotore und geeignete Getriebe vorgesehen, die von einer Bedienperson mittels einer ebenfalls nicht dargestellten Bedieneinheit betätigbar sind. Lediglich die zum Verstellen der Lagerungsplatte 1 relativ zu dem Basisteil 3 in Richtung der Längsachse des Patientenlagerungstisches (Doppelpfeil x) vorgesehene Antriebseinrichtung ist in Fig. 3 schematisch dargestellt. Sie besteht aus einem in dem Basisteil 3 angeordneten Elektromotor 54, der eine Gewindespindel 55 antreibt. Die Gewindespindel 55 wirkt mit einer Mutter 56 zusammen, die an der Lagerungsplatte 1 in nicht dargestellter Weise lösbar befestigt ist. Dabei ist in dem Basisteil 3 ein Schlitz vorgesehen, durch den die Mutter 56 mit der Lagerungsplatte 1 in Eingriff steht.

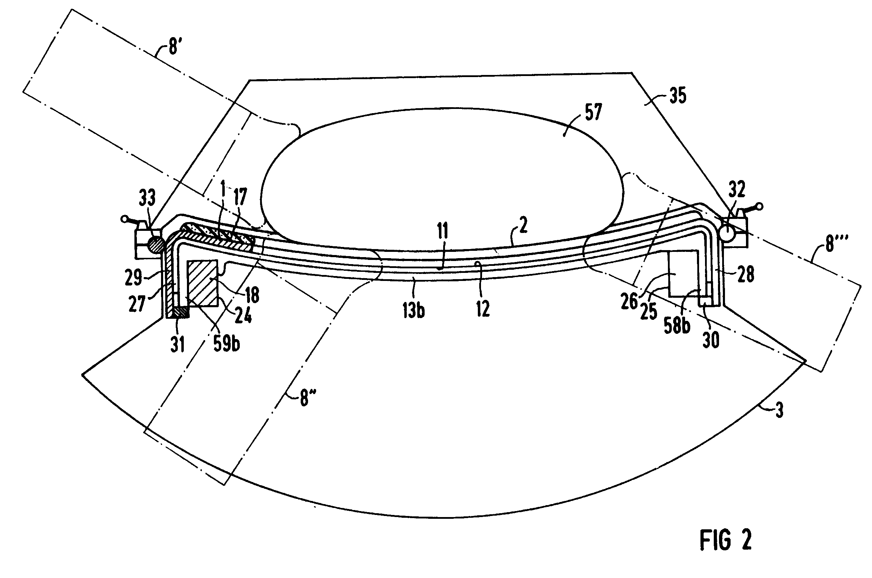

Aus den Fig. 1, 3 und 5 wird deutlich, daß der Patientenlagerungstisch mit seinem Basisteil 3 derart an dem Tragteil 4 angebracht ist, daß der Patientenlagerungstisch nur an seinem einen Ende unterstützt ist. Bei dem unterstützten Ende des Patientenlagerungstisches handelt es sich um dessen Fußende. Das freie Ende des Patientenlagerungstisches bildet dessen Kopfende. Infolge des Umstandes, daß der Patientenlagerungstisch nur an seinem einen Ende unterstützt ist, ergibt sich eine verbesserte Zugänglichkeit zu dem Patientenlagerungstisch bzw. einem auf diesem liegenden Patienten. Um eine an einer geeigneten, nicht dargestellten Halterung angebrachte Stoßwellenquelle 8, die in Fig. 1 schematisch angedeutet ist, aus möglichst beliebigen Richtungen an der Körperoberfläche eines auf der Lagerungsplatte 1 liegenden Patienten zur Anlage bringen zu können, ist die Lagerungsplatte 1 mit einem Ausschnitt versehen, der als etwa rechteckige Ausnehmung 9 in der in Fig. 1 vorderen Längskante der Lagerungsplatte 1 ausgeführt ist. Wie aus Fig. 2 anhand der strichliert dargestellten und mit 8′ und 8˝ bezeichneten Extrempositionen der Stoßwellenquelle 8 ersichtlich ist, ist nur ein relativ geringer Winkelbereich durch den im Bereich der Ausnehmung 9 verbleibenden Abschnitt 10 der Lagerungsplatte 1 versperrt. Insbesondere ist es möglich, die Stoßwellenquelle 8 von schräg unten lateral an den im Querschnitt angedeuteten Körper 57 eines auf der Lagerungsplatte 1 liegenden Patienten anzukoppeln. Diese Position der Stoßwellenquelle 8 ist ebenfalls strichliert dargestellt und mit 8‴ bezeichnet.

Um in Behandlungsfällen, die es erfordern, daß die Stoßwellenquelle 8 von der Unterseite des Patientenlagerungstisches her durch die Ausnehmung 9 mit dem Körper 57 des Patienten in Eingriff gebracht wird, eine Positionierung des Patienten relativ zu der Stoßwellenquelle 8 durch Verschieben der Lagerungsplatte 1 in Richtung der Längsachse des Patientenlagerungstisches bei bereits in der Ausnehmung 9 befindlicher Stoßwellenquelle 8 zu ermöglichen, weist die Ausnehmung 9 in Richtung der Längsachse des Patientenlagerungstisches eine lichte Weite a (Fig. 3) auf, die größer ist als dies die entsprechende Abmessung der Stoßwellenquelle 8, z.B. deren Durchmesser, erfordert. Es reicht zwar aus, wenn die Lagerungsplatte 1 bei in der Ausnehmung 9 befindlicher Stoßwellenquelle 8 in Richtung der Längsachse des Patientenlagerungstisches um etwa ± 100 mm verschiebbar ist; bei einem Durchmesser der Stoßwellenquelle von ca. 250 mm bedeutet dies jedoch, daß die Lagerungsplatte 1 allein keine ausreichende Unterstützung des Patienten im Bereich der dann eine lichte Weite a von ca. 450 mm aufweisenden Ausnehmung 9 bieten kann.

Um eine ausreichende Unterstützung des Patienten im Bereich der Ausnehmung 9 zu gewährleisten und insbesondere ein Durchrutschen des Patienten durch die Ausnehmung 9 zu vermeiden, weist das Basisteil 3 einen der Gestalt der Lagerungsplatte 1 im wesentlichen angepaßten plattenförmigen Abschnitt 13 auf, der derart unterhalb der Lagerungsplatte 1 angeordnet ist, daß seine Oberseite 11 der Unterseite 12 der Lagerungsplatte 1 zugewandt ist. Ähnlich wie die Lagerungsplatte 1 besitzt auch das Basisteil 3 einen Ausschnitt. Dieser ist ähnlich wie der Ausschnitt im Falle der Lagerungsplatte 1 als Ausnehmung 14 in der in Fig. 1 vorderen Längskante des plattenförmigen Abschnittes 13 ausgeführt. Die lichte Weite b der Ausnehmung 14 in Richtung der Längsachse des Patientenlagerungstisches ist wenig größer als der Durchmesser der Stoßwellenquelle 8 und ist somit deutlich kleiner als die lichte Weite a der Ausnehmung 9 der Lagerungsplatte 1.

Es versteht sich, daß bei von der Unterseite des Patientenlagerungstisches her mit dem Körper 57 eines Patienten in Eingriff stehender Stoßwellenquelle 8 die Lagerungsplatte 1 und das Basisteil 3 relativ zueinander so angeordnet sein müssen, daß die Ausnehmung 9 der Lagerungsplatte 1 die Ausnehmung 14 des plattenförmigen Abschnittes 13 vollständig freigibt, so wie dies in den Fig. 1 bis 3 und 5 dargestellt ist. Da die Lagerungsplatte 1, so wie dies aus den Fig. 1 bis 3 ersichtlich ist, mit den quer zur Längsachse des Patientenlagerungstisches und damit quer zur Verschieberichtung verlaufenden Begrenzungskanten 15 und 16 ihrer Ausnehmung 9 im wesentlichen spaltlos an der Oberseite 11 des plattenförmigen Abschnittes 13 anliegt, bietet die Oberseite 11 des plattenförmigen Abschnittes 13 eine wirksame zusätzliche Unterstützung des Körpers 57 des Patienten, die ein Durchrutschen des Patienten durch die Ausnehmung 9 der Lagerungsplatte 1 ausschließt. Der innerhalb der Ausnehmung 9 der Lagerungsplatte 1 befindliche Teil der Oberseite 11 des plattenförmigen Abschnittes 13 des Basisteiles 3 bildet also eine zusätzliche Auflagefläche für den Körper 57 des Patienten.

Wie aus den Figuren ersichtlich ist, ist die Auflagefläche der Lagerungsplatte mit einer Polsterauflage 17 versehen, die bis an die Begrenzungskanten der Ausnehmung 9 heranreicht. Somit ist, so wie dies aus der Fig. 3 ersichtlich ist, sichergestellt, daß unmittelbar im Bereich der Begrenzungskanten 15 und 16 der Ausnehmung 9 der Lagerungsplatte 1 ein Abstand der Körperoberfläche des Patienten von der Oberseite 1 des plattenförmigen Abschnittes 13 des Basisteiles 3 vorliegt, durch den bei Verschiebungen der Lagerungsplatte 1 relativ zu dem Basisteil 3 ein Einquetschen von Hautfalten zwischen der Lagerungsplatte 1 und dem plattenförmigen Abschnitt 13 vermieden ist.

Die Fig. 1 zeigt in Verbindung mit der Fig. 3, daß der plattenförmige Abschnitt des Basisteiles 3 zwei Plattenteile 13a, 13b aufweist, die in Richtung der Längsachse des Patientenlagerungstisches und damit in Verschieberichtung gesehen auf unterschiedlichen Seiten der Ausnehmung 14 des Basisteiles 3 angeordnet sind. Die Plattenteile 13a, 13b, deren einander zugewandte Kanten 52, 53 die Ausnehmung 14 begrenzen, sind durch einen Tragholm 18 miteinander verbunden. Der Tragholm 18 verläuft unterhalb des Bereiches 10 der Lagerungsplatte 1 entlang deren von der Ausnehmung 9 unberührten, also der in Fig. 1 hinteren, Längskante. Das Plattenteil 13b ist mit dem Basisteil 3 verbunden. Der Tragholm 18 und das an dem Tragholm 18 angebrachte Plattenteil 13a bilden also eine wirksame Unterstützung der durch die Ausnehmung 9 geschwächten Lagerungsplatte 1 im Bereich ihres freien Endes. Das Plattenteil 13a wirkt als an dem freien Ende des Tragholmes 18 angebrachter, sich quer zur Längsachse der Lagerungsplatte 1 erstreckender Tragarm, der Verwindungen der Lagerungsplatte 1 entgegenwirkt.

Anhand der Fig. 4 wird deutlich, daß das Plattenteil 13a an seinem zur Befestigung an dem Tragholm 18 vorgesehenen Ende einen etwa U-förmigen Abschnitt 19 aufweist, der den Tragholm 18, der einen etwa rechteckigen Querschnitt besitzt, von oben umgreift. Der äußere Schenkel 20 des U-förmigen Abschnittes 19 ist mit Langlöchern 21 versehen, in die bei an dem Tragholm 18 angebrachtem Plattenteil 13a an der äußeren Seitenfläche des Tragholmes 18 angebrachte Vorsprünge 22 eingreifen. Dabei liegen die Vorsprünge 22 an den unteren Begrenzungskanten der Langlöcher 21 an. Das Plattenteil 13a kann also von dem Tragholm getrennt werden, indem es an seinem freien Ende so weit angehoben wird, daß der innere Schenkel 23 seines U-förmigen Abschnittes 19 nicht mehr mit der inneren Seitenfläche des Tragholmes 18 in Eingriff steht, und indem es dann nach außen abgenommen wird. In entsprechender Weise kann das Plattenteil 13a auch wieder an dem Tragholm 18 angebracht werden. Es versteht sich, daß zum Abnehmen bzw. Anbringen des Plattenteiles 13a die Lagerungsplatte 1 von dem Basisteil 3 getrennt werden muß.

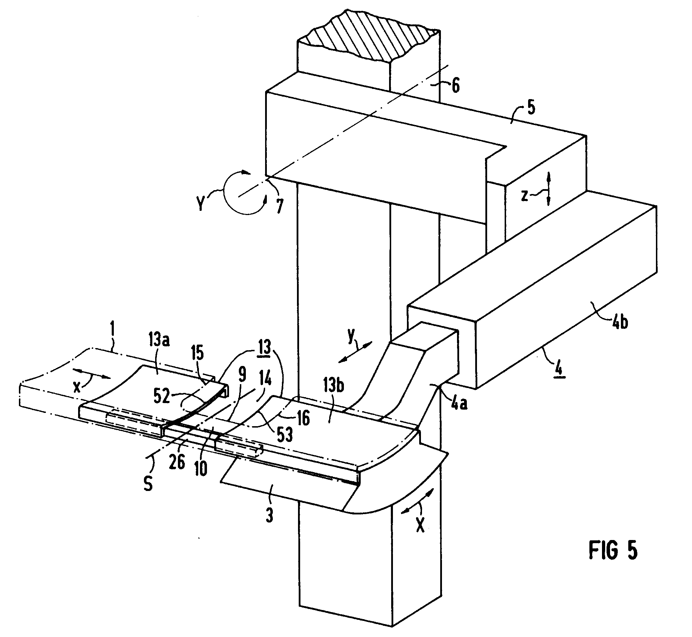

Wie insbesondere die Fig. 3 zeigt, ist der Tragholm 18 in einer entsprechenden Führungsbohrung 24 des Basisteiles 3 aufgenommen. In einer weiteren, auf der anderen Seite des Basisteiles 3 befindlichen Führungsbohrung 25 (siehe Fig. 2 und 5) ist ein weiterer Tragholm 26 aufgenommen. Die Tragholme 18, 26 sind in ihrer jeweiligen Führungsbohrung 24, 25 längsverschieblich geführt, so daß die Möglichkeit besteht, wahlweise einen der Tragholme 18, 26 aus dem Basisteil 3 auszuziehen und das Plattenteil 13a an dem jeweils ausgezogenen Tragholm 18 bzw. 26 anzubringen. Es besteht somit die Möglichkeit, den Patientenlagerungstisch ausgehend von dem Zustand gemäß Fig. 1, in dem sich die Ausnehmungen 9 und 14 in der vorderen Längskante des Patientenlagerungstisches befinden, derart zu verändern, daß sich die Ausnehmungen 9 und 14 gemäß Fig. 5 in der hinteren Längskante des Patientenlagerungstisches befinden. Hierzu ist es lediglich erforderlich, die Lagerungsplatte 1 von dem Basisteil 3 zu trennen, das Plattenteil 13a von dem Tragholm 18 zu trennen, den Tragholm 18 in die Führungsbohrung 24 einzuschieben, den Tragholm 26 aus seiner Führungsbohrung 25 auszuziehen, das Plattenteil 13a an dem Tragholm 26 anzubringen und die gewendete Patientenlagerungsplatte 1 wieder auf das Basisteil 3 aufzusetzen.

Um sicherzustellen, daß sich beim Wenden der Lagerungsplatte 1 die Lage der Ausnehmung 9 der Lagerungsplatte 1 in bezug auf die Längsachse des Patientenlagerungstisches nicht ändert, ist die Lagerungsplatte 1 zu einer quer zur Längsachse des Patientenlagerungstisches und durch die Ausnehmung 9 verlaufende Symmetrieachse S spiegelsymmetrisch ausgebildet.

Um die erwähnte Verstellbarkeit der Lagerungsplatte 1 relativ zu dem Basisteil 3 zu gewährleisten, ist die von der Auflagefläche 2 abgewandte Unterseite 12 der Lagerungsplatte 1 als Eingriffsfläche ausgebildet, die an der ebenfalls als Eingriffsfläche ausgebildeten Oberseite 11 des plattenförmigen Abschnittes 13 des Basisteiles 3 flächenhaft anliegt. Wird die Lagerungsplatte 1 relativ zu dem Basisteil 3 verstellt, gleiten die Unterseite 12 der Lagerungsplatte 1 und die Oberseite 11 des plattenförmigen Abschnittes 13 des Basisteiles 3 aufeinander. Die Lagerungsplatte 1 und der plattenförmige Abschnitt 13 des Basisteiles 3 sind einander nicht nur hinsichtlich ihrer äußeren Abmessungen, d.h. ihrer Länge und Breite, sondern auch in ihrem Querschnitt weitgehend angepaßt, so daß sich zwischen der Unterseite 11 der Lagerungsplatte 1 und der Oberseite 13 des plattenförmigen Abschnittes 13 des Basisteiles 3 eine große Anlagefläche ergibt. Die zwischen den beiden Teilen vorliegende Flächenpressung ist somit nur gering, so daß kein wesentlicher Verschleiß auftreten kann. Ebenfalls im Interesse eines geringen Verschleißes ist die Oberseite 11 des plattenförmigen Abschnittes 13 des Basisteiles 3 durch einen reibungsmindernden Belag 27 gebildet, wobei dann die der Unterseite 12 der Lagerungsplatte 1 zugewandte Seite des Belages 27 die eigentliche Eingriffsfläche des Basisteiles 3 bildet. Der reibungsmindernde Belag 27 bewirkt zugleich, daß für die Verstellung der Lagerungsplatte 1 nur eine geringe Betätigungskraft erforderlich ist. Als Material für den reibungsmindernden Belag ist ein polymerer Werkstoff, nämlich Polyäthylen vorgesehen, wodurch der Belag 27 auf einfache Weise, nämlich durch Kleben, an dem Basisteil 3 befestigt ist. Um die Mutter 56 in der erforderlichen Weise an der Lagerungsplatte 1 befestigen zu können, ist der plattenförmige Abschnitt 13 mit einem Schlitz 61 versehen, der sich durch das Plattenteil 13b und den Belag 27 erstreckt und sich mit dem Schlitz 60 des Basisteiles 3 deckt. Die Breite der Schlitze 60 und 61 ist auf das zum Durchtritt der Mutter 56 erforderliche Maß beschränkt.

Infolge der beschriebenen Ausbildung der Unterseite 12 der Lagerungsplatte 1 und der Oberseite 11 des plattenförmigen Abschnittes 13 des Basisteiles 3 als Eingriffsflächen erfährt die als dünnwandiges schalenförmiges Bauteil ausgebildete Lagerungsplatte 1 eine gleichmäßige Unterstützung, so daß Verwindungen und Durchbiegungen der Lagerungsplatte 1 nicht zu befürchten sind. Die Lagerungsplatte 1 ist aus faserverstärktem Kunststoff, vorzugsweise kohlefaserverstärktem Kunststoff, gebildet. Es ist dann auf einfache Weise möglich, die Unterseite 12 der Lagerungsplatte 1 unmittelbar auf spanlosem Wege bei der Herstellung der Lagerungsplatte 1 als Eingriffsfläche auszubilden. Auch der plattenförmige Abschnitt 13 des Basisteiles 3 ist aus faserverstärktem Kunststoff, vorzugsweise kohlefaserverstärktem Kunststoff, gebildet, so daß auch hier die Oberfläche des plattenförmigen Abschnittes 13 bereits bei dessen Herstellung durch spanlose Formgebung die Form erhält, die erforderlich ist, um nach dem Aufkleben des Belages 27 eine Oberseite 11 zu erhalten, die geeignet ist, um mit der Unterseite 12 der Lagerungsplatte 1 wie beschrieben zusammenwirken zu können.

Die Verstellbarkeit der Lagerungsplatte 1 in Längsrichtung des Patientenlagerungstisches beschränkt sich nicht auf die erwähnten ± 100 mm. Vielmehr ist, um beispielsweise endoskopische Untersuchungen durchführen zu können, eine Verstellbarkeit in Längsrichtung des Patientenlagerungstisches von insgesamt etwa 700 mm gegeben. Aus diesem Grunde ist die gesamte Unterseite 12 der Lagerungsplatte 1 als Eingriffsfläche ausgebildet. Im Falle des dargestellten Ausführungsbeispieles beträgt die Größe der Eingriffsfläche, d.h. der Oberseite 11, des plattenförmigen Abschnittes 13 des Basisteiles 3 etwa 60 % der Fläche der Eingriffsfläche der Lagerungsplatte 1. Da in beiden Extrempositionen, die die Lagerungsplatte 1 in bezug auf das Basisteil 3 einnehmen kann, die gesamte Oberseite 11 des plattenförmigen Abschnittes 13 des Basisteiles 3 an der Unterseite 12 der Lagerungsplatte 1 anliegt, ist stets eine gute Unterstützung der Lagerungsplatte 1 gewährleistet. Für den Fall, daß nicht die gesamte Unterseite 12 der Lagerungsplatte 1 als Eingriffsfläche ausgebildet ist, sollte diese wenigstens 30 % der Fläche der Unterseite 12 der Lagerungsplatte 1 umfassen. Außerdem sollte sichergestellt sein, daß stets wenigstens 50 % der Unterseite 12 der Lagerungsplatte 1 an der Eingriffsfläche des Basisteiles 3 anliegen. Die Eingriffsflächen sowohl der Lagerungsplatte 1 als auch das Basisteiles 3 müssen nicht unbedingt als durchgehende Flächen ausgebildet sein, sondern können in mehrere Abschnitte unterteilt sein, so wie dies beispielsweise im Falle der Oberseite 11 des plattenförmigen Abschnittes 13 des Basisteiles 3 infolge des Umstandes der Fall ist, daß der plattenförmige Abschnitt 13 aus den beiden Plattenteilen 13a, 13b zusammengesetzt ist.

Die Eingriffsflächen sowohl der Lagerungsplatte 1 als auch des plattenförmigen Abschnittes 13 des Basisteiles 3 erstrecken sich jeweils bis an die quer zur Tischlängsrichtung verlaufenden Begrenzungskanten 15, 16 der Ausnehmung 9 bzw. die Begrenzungskanten 52, 53 der Ausnehmung 14. Hierdurch ist die bereits erwähnte im wesentlichen spaltlose Anlage der Begrenzungskanten 15, 16 der Ausnehmung 9 an der Oberseite 11 des plattenförmigen Abschnittes 13 des Basisteiles 3 gewährleistet.

Um eine sichere Führung der Lagerungsplatte 1 relativ zu dem Basisteil 3 quer zur Längsachse des Patientenlagerungstisches, also quer zur Verschieberichtung, zu gewährleisten, ist die Lagerungsplatte an ihren beiden Längskanten mit nach unten abgewinkelten Führungsborden 28, 29 versehen, die mit ihren Innenseiten an den entsprechenden ebenfalls nach unten abgewinkelten Längskanten 58a, 59a bzw. 58b, 59b der Plattenteile 13a und 13b des plattenförmigen Abschnittes 13 des Basisteiles 3 anliegen. Dabei stellen die Innenseiten der Führungsborde 28, 29 Bestandteile der Eingriffsfläche der Lagerungsplatte 1 und die Außenseiten der nach unten abgewinkelten Längskanten 58a, 59a, 58b, 59b der Plattenteile 13a, 13b Bestandteile der Eingriffsfläche des Basisteiles 3 dar. Der Belag 27 bedeckt daher auch die Außenseiten der Längskanten 58a, 59a, 58b, 59b. Der an der mit der Ausnehmung 9 versehenen Längskante der Lagerungsplatte 1 angebrachte Führungsbord 28 weist im Bereich der Ausnehmung eine Unterbrechung auf. Die Führungsborde 28, 29 sind an den Innenseiten ihrer freien Enden mit Leisten 30, 31 versehen, die von unten an den abgewinkelten Längskanten der Plattenteile 13a, 13b des plattenförmigen Abschnittes 13 des Basisteiles 3 anliegen, wobei die Leiste 30 im Bereich der Ausnehmung 9 der Lagerungsplatte 1 unterbrochen ist. Es ist so ein sicherer Zusammenhalt der Lagerungsplatte 1 mit dem Basisteil 3 insbesondere auch beim Schwenken des Patientenlagerungstisches in Richtung des gekrümmten Doppelpfeiles X und/oder des gekrümmten Doppelpfeiles Y gewährleistet.

Um die Patientenlagerungsplatte in der zuvor beschriebenen Weise wenden zu können, muß diese also von der zur Verschiebung der Lagerungsplatte 1 in Richtung des Doppelpfeiles x dienenden Antriebseinrichtung 54, 55, 56 getrennt werden und in Richtung der Längsachse des Patientenlagerungstisches von dem Basisteil 3 abgezogen werden. Die gewendete Lagerungsplatte 1 wird anschließend in umgekehrter Richtung wieder auf das Basisteil 3 aufgeschoben und mit der erwähnten Antriebseinrichtung gekoppelt. Zur Trennung der Antriebseinrichtung 54, 55, 56 von der Lagerungsplatte 1 wird die Mutter 56 von der Lagerungsplatte 1 gelöst, indem eine nicht dargestellte, von der Oberseite der Lagerungsplatte her zugängliche Schraube gelöst wird, die zur Befestigung der Mutter 56 an der Lagerungsplatte 1 dient.

Die Lagerungsplatte 1 ist mit entlang ihren Längskanten verlaufenden Schienen 32, 33 kreisförmigen Querschnittes versehen, die zur Befestigung von Zubehörteilen dienen. Dabei ist die an der mit der Ausnehmung 9 versehenen Längskante der Lagerungsplatte 1 angebrachte Schiene 32 im Bereich der Ausnehmung 9 unterbrochen. Die Schienen 32, 33 dienen zur Befestigung von Zubehörteilen, so wie dies in Fig. 1 am Beispiel eines Infusions-Ständers 34 und einer Fußbank 35 angedeutet ist. Der Infusions-Ständer 34 und die Fußbank 35 sind mit geeigneten Klemmvorrichtungen versehen, die eine Befestigung dieser Teile an einer gewünschten Position längs der Lagerungsplatte 1 gestatten.

Wie aus der Fig. 1 ersichtlich ist, besteht die Möglichkeit, die Ausnehmung 9 der Lagerungsplatte 1 mittels eines Verschlußteiles 36, das von den Plattenteilen 13a und 13b des plattenförmigen Abschnittes 13 des Basisteiles 3 unterstützt wird, zu verschließen, indem das Verschlußstück 36 in die Ausnehmung 9 eingelegt wird. Das Verschlußstück 36 ist mit einer Polsterauflage 37 versehen, deren Dicke der der Polsterauflage 17 entspricht.

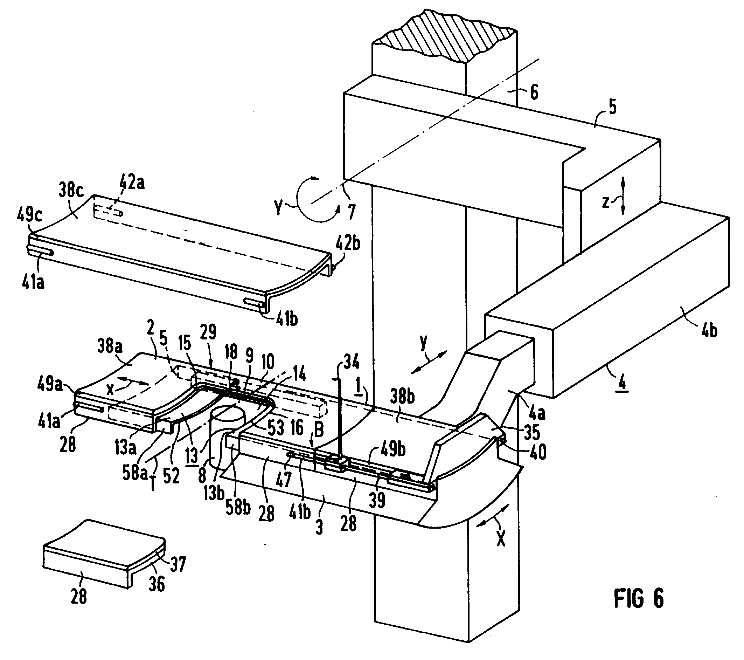

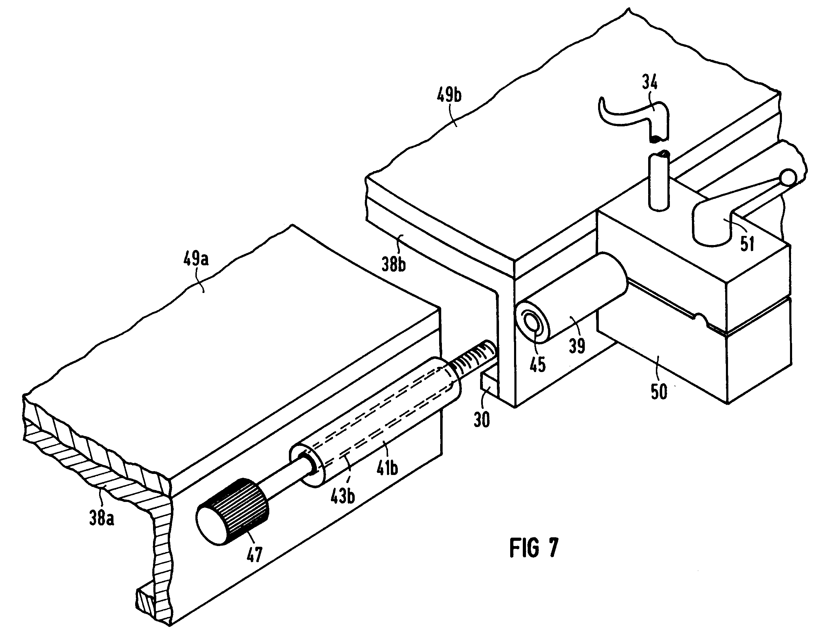

In den Fig. 6 und 7 ist ein erfindungsgemäßer Patientenlagerungstisch dargestellt, der sich von dem zuvor beschriebenen, nicht zur Erfindung gehörigen Patientenlagerungstisch durch die Ausbildung der Lagerungsplatte 1 unterscheidet, weshalb in den Fig. 6 und 7 für gleiche Teile die gleichen Bezugszeichen wie in den vorangegangenen Figuren verwendet werden. Im Falle der Fig. 6 ist die Lagerungsplatte 1 zweiteilig ausgeführt und weist die miteinander verbundenen Plattenabschnitte 38a und 38b auf. Dabei ist der das freie Ende des Patientenlagerungstisches bildende Plattenabschnitt 38a mit der Ausnehmung 9 versehen. Die nicht dargestellte zur Verschiebung der Lagerungsplatte 1 in Richtung des Doppelpfeiles x dienende Antriebseinrichtung greift mit ihrer Mutter an dem an dem Basisteil 3 angebrachten Plattenabschnitt 38b an. Der Plattenabschnitt 38b ist mit Schienen 39, 40 versehen, die entlang seiner Längskanten verlaufen und einen kreisförmigen Querschnitt aufweisen. Die Schienen 39, 40 dienen zur Befestigung von Zubehörteilen, z.B. eines Infusions-Ständers 34, sowie zur Befestigung der Fußbank 35. Im Bereich der Enden des Plattenabschnittes 38a ist dieser an seinen Längskanten 28, 29 mit Schienenabschnitten versehen, von denen in Fig. 6 nur die Schienenabschnitte 41a, 41b sichtbar sind. Die Schienenabschnitte besitzen, so wie Fig. 7 dies für den Schienenabschnitt 41b anhand der Bohrung 43b zeigt, axial durchgehende Bohrungen. An ihren von dem Basisteil 3 abgewandten Enden sind die Schienen 39, 40 in der aus Fig. 7 am Beispiel der Schiene 39 und der Gewindebohrung 45 ersichtlichen Weise mit Gewindebohrungen versehen, die bei auf dem plattenförmigen Abschnitt 13 des Basisteiles 3 aufgesetztem Plattenabschnitt 38a mit den Bohrungen der Schienenabschnitte fluchten. Wie dies aus Fig. 7 am Beispiel des Schienenabschnittes 41b mit der Bohrung 43b, der Schiene 39 mit der Gewindebohrung 45 und der Rändelschraube 47 ersichtlich ist, sind die beiden Plattenabschnitte 38a, 38b der Lagerungsplatte 1 mit Hilfe von Rändelschrauben, die sich durch die Bohrungen der Schienenabschnitte erstrecken und in die Gewindebohrungen der Schienen 39, 40 einschraubbar sind, lösbar miteinander verbunden.

Werden die Rändelschrauben entfernt, kann der Plattenabschnitt 38a axial von dem plattenförmigen Abschnitt 13 des Basisteiles 3 abgezogen, gewendet und wieder auf den plattenförmigen Abschnitt 13 des Basisteiles 3 aufgeschoben werden, so daß sich die in dem Plattenabschnitt 38a der Lagerungsplatte 1 befindliche Ausnehmung 9 wahlweise in der hinteren oder der vorderen Längskante der Lagerungsplatte 1 befinden kann. Es versteht sich, daß dann jeweils nur der geeignete Tragholm 18 bzw. 26 aus dem Basisteil 3 ausgezogen und mit dem Plattenteil 13a versehen ist. Im Gegensatz zu dem zuvor beschriebenen Ausführungsbeispiel erübrigt sich infolge der Teilung der Lagerungsplatte 1 in die zwei Plattenabschnitt 38a, 38b beim Wenden des Plattenabschnittes 38a die Trennung der erwähnten Antriebseinrichtung von der Lagerungsplatte 1. Um sicherzustellen, daß sich beim Wenden des Plattenabschnittes 38a die Lage der Ausnehmung 9 in bezug auf die Längsachse des Patientenlagerungstisches nicht ändert, ist der Plattenabschnitt 38a ähnlich wie im Falle des zuvor beschriebenen Ausführungsbeispieles die Lagerungsplatte 1 zu einer quer zur Längsachse des Patientenlagerungsrungstisches und durch die Ausnehmung 9 verlaufenden Symmetrieachse T spiegelsymmetrisch ausgebildet.

Ähnlich wie im Falle des zuvor beschriebenen Ausführungsbeispieles besteht die Möglichkeit, die Ausnehmung 9 des Plattenabschnittes 38a, beide Plattenabschnitte 38a und 38b sind übrigens mit Polsterauflagen 49a bzw. 49b versehen, durch das mit der Polsterauflage 37 versehene Verschlußstück 36 zu verschließen. Außerdem besteht, so wie dies in Fig. 6 angedeutet ist, für Behandlungsfälle, die dies gestatten, die Möglichkeit, anstelle des mit der Ausnehmung 9 versehenen Plattenabschnittes 38a einen ohne Ausnehmung ausgeführten Plattenabschnitt 38c mit dem Plattenabschnitt 38b zu verbinden. Der Plattenabschnitt 38c ist mit einer Polsterauflage 49c versehen. Im Falle des Plattenabschnittes 38c sind übrigens sämtliche vier Schienenabschnitte 41a, 41b, 42a, 42b dargestellt.

Aus der Fig. 7 ist ersichtlich, daß der Infusions-Ständer 34 mit einem klammerartigen Klemmstück 50 versehen ist, das mittels einer Klemmschraube 51 auf der Schiene 39 klemmbar ist. Die Befestigung der Fußbank 35 erfolgt in entsprechender Weise (nicht dargestellt). Auch die Befestigung des Infusionsständers 34 und der Fußbank 35 im Falle des zuerst beschriebenen Ausführungsbeispieles an den Schienen 32, 33 erfolgt in der in Fig. 7 verdeutlichten Weise.

Die Erfindung wurde vorstehend am Beispiel eines Patientenlagerungstisches beschrieben, der Bestandteil eines Arbeitsplatzes zur Behandlung eines Patienten mit fokussierten Stoßwellen zur nicht invasiven Zertrümmerung von im Körper des Patienten befindlichen Konkrementen ist. Es können jedoch auch Röntgenuntersuchungstische, Patientenlagerungstische für die Urologie, Lagerungstische für die Strahlentherapie usw. gemäß der Erfindung ausgebildet werden. Außerdem ist im Falle des vorstehend beschriebenen erfindungsgemäßen Patientenlagerungstisches nur eine Verstellbarkeit der Lagerungsplatte 1 relativ zu dem Basisteil 3 vorgesehen, die in Richtung der Längsachse des Patientenlagerungstisches und geradlinig erfolgt. Mit den Merkmalen der Erfindung sind jedoch auch andere und/oder zusätzliche Verstellmöglichkeiten realisierbar.

## Patentansprüche

1. Patientenlagerungstisch mit einer Lagerungsplatte (1), welche einen Ausschnitt aufweist, durch den die Körperoberfläche eines auf der Lagerungsplatte (1) liegenden Patienten für medizinische Zwecke zugänglich ist, wobei der Ausschnitt als Ausnehmung (9) in einer der Begrenzungskanten der Lagerungsplatte (1) ausgeführt ist, **dadurch gekennzeichnet,** daß der Patientenlagerungstisch ein Basisteil (3) aufweist, an dem die Lagerungsplatte (1) anbringbar ist, und daß die Lagerungsplatte (1) zweiteilig ausgeführt ist, wobei ein Plattenabschnitt (38a) die Ausnehmung (9) aufweist und mit dem anderen Plattenabschnitt (38b) wahlweise derart verbindbar ist, daß sich die Ausnehmung (9) auf der einen oder der anderen Seite des Patientenlagerungstisches befindet.

2. Patientenlagerungstisch nach Anspruch 1, **dadurch gekennzeichnet,** daß der die Ausnehmung (9) aufweisende Plattenabschnitt (38a) zu einer quer zu der mit der Ausnehmung (9) versehenen Begrenzungskante durch die Ausnehmung (9) verlaufenden Symmetrieachse (S, T) spiegelsymmetrisch ausgebildet ist.

3. Patientenlagerungstisch nach Anspruch 1 oder 2, **dadurch gekennzeichnet,** daß eine der Längskanten der Lagerungsplatte (1) mit der Ausnehmung (9) versehen ist.

4. Patientenlagerungstisch nach einem der Ansprüche 1 bis 3, **dadurch gekennzeichnet,** daß der Patientenlagerungstisch ein freies Ende aufweist und mit seinem anderen Ende an einem Tragteil (4) angebracht ist.

5. Patientenlagerungstisch nach einem der Ansprüche 1 bis 4, **dadurch gekennzeichnet,** daß ein Tragholm (18, 26) zur Unterstützung der Lagerungsplatte (1) vorgesehen ist, der sich wenigstens im Bereich der Ausnehmung (9) entlang der der Ausnehmung (9) gegenüberliegenden Begrenzungskante der Lagerungsplatte (1) erstreckt.

6. Patientenlagerungstisch nach den Ansprüchen 4 und 5, **dadurch gekennzeichnet,** daß der Tragholm (18, 26) ein freies Ende aufweist, an dem ein sich quer zur Längsachse des Tragholmes (18, 26) erstreckender Tragarm (13a) anbringbar ist, der die Lagerungsplatte (1) im Bereich des freien Endes des Patientenlagerungstisches unterstützt.

7. Patientenlagerungstisch nach Anspruch 5 oder 6, **dadurch gekennzeichnet,** daß zwei Tragholme (18, 26) vorgesehen sind, die in Abhängigkeit von der Position der Ausnehmung (9) auf der einen oder der anderen Seite des Patientenlagerungstisches wahlweise ausziehbar sind.

8. Patientenlagerungstisch nach Anspruch 7, **dadurch gekennzeichnet,** daß die Tragholme (18, 26) aus dem Basisteil (3) ausziehbar sind.

## Claims

1. Patient support table having a support plate (1) which has a cut-out through which the body surface of a patient lying on the support plate (1) is accessible for medical purposes, wherein the cut-out is formed as a recess (9) in one of the boundary edges of the support plate (1), characterised in that the patient support table has a base part (3) to which the support plate (1) can be attached, and in that the support plate (1) is constructed in two parts, wherein a plate section (38a) has the recess (9) and can be selectively connected to the other plate section (38b) in such a way that the recess (9) is located on either side of the patient support table.

2. Patient support table according to claim 1, characterised in that the plate section (38a) having the recess (9) is constructed so that it is mirror-symmetrical about an axis of symmetry (S, T) which extends through the recess (9) transversely relative to the boundary edge provided with the recess (9).

3. Patient support table according to claim 1 or 2, characterised in that one of the longitudinal edges of the support plate (1) is provided with the recess (9).

4. Patient support table according to one of claims 1 to 3, characterised in that the patient support table has a free end and is attached at its other end to a carrying member (4).

5. Patient support table according to one of claims 1 to 4, characterised in that a carrying spar (18, 26) which extends at least in the region of the recess (9) along the boundary edge of the support plate (1) located opposite the recess (9), is provided for supporting the support plate (1).

6. Patient support table according to claims 4 and 5, characterised in that the carrying spar (18, 26) has a free end to which there can be attached a bracket (13a) extending transversely relative to the longitudinal axis of the carrying spar (18, 26), which bracket supports the support plate (1) in the region of the free end of the patient support table.

7. Patient support table according to claim 5 or 6, characterised in that there are provided two carrying spars (18, 26) which can be selectively pulled out in dependence upon the position of the recess (9) on either side of the patient support table.

8. Patient support table according to claim 7, characterised in that the carrying spars (18, 26) can be pulled out from the base part (3).

## Revendications

1. Table de support pour patients comportant un plateau formant couchette (1), qui possède une découpe, par laquelle la surface du corps d'un patient allongé sur le plateau formant couchette (1) est accessible à des fins médicales, la découpe étant réalisée sous la forme d'une ouverture (9) ménagée dans l'un des bords limites du plateau formant couchette (1), caractérisée par le fait que la table de support pour patients comporte une partie de base (3), sur laquelle peut être monté le plateau formant couchette (1), et que le plateau formant couchette (1) est réalisé en deux éléments, une section (38a) du plateau comportant l'ouverture (9) et pouvant être raccordée au choix à l'autre section (38b) du plateau de telle sorte que l'ouverture (9) est située d'un côté ou de l'autre côté de la table de support pour patients.

2. Table de support pour patients suivant la revendication 1, caractérisée par le fait que la section (38a) du plateau qui possède l'ouverture (9), est agencée de manière à être symétrique par rapport à un axe de symétrie (S,T) qui s'étend transversalement à travers l'ouverture (9) par rapport aux bords limites formés par l'ouverture (9).

3. Table de support pour patients suivant la revendication 1 ou 2, caractérisée par le fait que l'un des bords longitudinaux du plateau formant couchette (1) possède l'ouverture (9).

4. Table de support pour patients suivant l'une des revendications 1 à 3, caractérisée par le fait que la table de support pour patients possède une extrémité libre et est montée, par son autre extrémité, sur une partie de support (4).

5. Table de support pour patients suivant l'une des revendications 1 à 4, caractérisée par le fait qu'il est prévu un longeron (18,26) servant à supporter le plateau formant couchette (1) et qui s'étend au moins dans la zone de l'ouverture (9), le long du bord limite du plateau formant couchette (1), situé à l'opposé de l'ouverture (9).

6. Table de support pour patients suivant les revendications 4 et 5, caractérisée par le fait que le longeron (18,26) possède une extrémité libre, sur laquelle peut être monté un bras de support (13a), qui s'étend transversalement par rapport à l'axe longitudinal du longeron (18,26) et qui supporte le plateau formant couchette (1) dans la zone de l'extrémité libre de la table de support pour patients.

7. Table de support pour patients suivant la revendication 5 ou 6, caractérisée par le fait qu'il est prévu deux longerons (18,26), qui peuvent être ressortis au choix en fonction de la position de l'ouverture (9), d'un côté ou de l'autre du plateau de support pour patients.

8. Table de support pour patients suivant la revendication 7, caractérisée par le fait que les longerons (18,26) peuvent être retirés de la partie de base (3).
